Europäisches Patentamt

European Patent Office

Office européen des brevets

(19)

(11) Veröffentlichungsnummer : **0 170 650**
**B1**

(12)

# EUROPÄISCHE PATENTSCHRIFT

(45) Veröffentlichungstag der Patentschrift :
04.05.88

(21) Anmeldenummer : 84900967.5

(22) Anmeldetag : 30.01.84

(86) Internationale Anmeldenummer :
PCT/DE 84/00024

(87) Internationale Veröffentlichungsnummer :
WO/8503217 (01.08.85 Gazette 85/17)

(51) Int. Cl.⁴ : **A 61 F   9/00, A 61 B 17/32**

(54) GERÄT ŻUM PERFORIEREN DER LINSENKAPSELVORDERWAND IM AUGE VON LEBEWESEN.

(43) Veröffentlichungstag der Anmeldung :
12.02.86 Patentblatt 86/07

(45) Bekanntmachung des Hinweises auf die Patenterteilung : 04.05.88 Patentblatt 88/18

(84) Benannte Vertragsstaaten :
FR GB NL

(56) Entgegenhaltungen :
DE-A- 3 038 024
DE-A- 3 205 959
FR-A- 2 211 207
US-A- 4 061 146
US-A- 4 246 902

(73) Patentinhaber : SCHLEGEL, Hans-Joachim
Siebenpfeifferstrasse 22
D-6650 Homburg/Saar (DE)

(72) Erfinder : SCHLEGEL, Hans-Joachim
Siebenpfeifferstrasse 22
D-6650 Homburg/Saar (DE)

(74) Vertreter : Wey, Hans-Heinrich, Dipl.-Ing. et al
Patentanwälte Müller-Börner & Wey Widenmayerstrasse 49
D-8000 München 22 (DE)

EP 0 170 650 B1

## Beschreibung

Die Erfindung betrifft ein Gerät zum Perforieren der Linsenkapselvorderwand im Auge von Lebewesen zwecks Entfernens eines Teilstücks der Kapselwand und Herstellung einer Öffnung in dieser, durch welche hindurch der Linsenkapselinhalt entfernbar ist, mit einem an einem Handstück angeordneten Röhrchen.

Es war bisher üblich, den Linsenkapselsack mittels verschiedenster Instrumente zu eröffnen (siehe z. B. DE-A-3 038 024 und DE-A-3 205 959), wobei jedoch in aller Regel der Rand der in die Vorderwand des Linsenkörpersacks geschnittenen Öffnung eine mehr oder weniger unregelmäßig gezackte oder ausgefranste Form aufweist. Dies ist jedoch ungünstig, speziell im Hinblick auf operative Maßnahmen, um dem Patienten eine Implantatlinse einzusetzen.

Es besteht schon seit langem der Wunsch, über ein Gerät verfügen zu können, welches in der Lage ist, aus der Vorderwand des Linsenkapselsacks eine kreisrunde Scheibe bestimmter Größe auszuschneiden, um eine kreisrunde Öffnung mit glattem, ungezacktem Rand längs der Schnittlinie zu erhalten. Geräte, die dies ermöglichen, sind bisher nicht bekanntgeworden, weshalb der Erfindung die Aufgabe zugrundeliegt, ein solches Gerät zu schaffen, welches die vorstehend aufgezeigte Aufgabe zu erfüllen vermag.

Zur Lösung dieser Aufgabe wurde vorgeschlagen, das in Betracht kommende Gerät in der Weise auszubilden, daß sich an bzw. in einem in das eröffnete Auge einführbaren Schneidkopf eine ringförmige Führungsbahn befindet, entlang welcher ein diese in axialer Richtung überragendes, bei seiner Bewegung einen Kreisschnitt ausführendes Schneidmesser geführt ist.

Das vorerwähnte Gerät erfüllt zwar seine Aufgabe in durchaus zufriedenstellender Weise, jedoch ist es durch seinen mechanischen Aufbau bedingt, außerordentlich empfindlich und damit störanfällig. Daher ist es als ein bei den in Betracht kommenden Operationen einzusetzendes Gerät ungeeignet.

Der Erfindung liegt die Aufgabe zugrunde, ein für den erwähnten Zweck geeignetes, konstruktiv einfaches und zuverlässig arbeitendes, stets einsatzbereites Gerät zu schaffen, welches es ermöglicht, die Linsenkapsel rasch und zuverlässig in gewünschter, optimaler Weise zu eröffnen, so daß ein Loch in der Vorderwand erhalten wird, das sich an der Stelle befindet, an der es gewünscht wird, und welches hinsichtlich seiner Größe und Form definiert ist. Dabei soll weiterhin während des Einsatzes dieses Geräts der hydrostatische Druck in der Vorderkammer des Auges aufrechterhalten bleiben, um in einem optimalen Operationsfeld arbeiten zu können.

Zur Lösung dieser Aufgabe wird gemäß der Erfindung vorgeschlagen, das in Betracht kommende Gerät in der Weise auszubilden, wie dies im Patentanspruch 1 und weiterhin in den Unteransprüchen angegeben ist, sowie in Bezug auf ein bevorzugtes Ausführungsbeispiel beschrieben und dargestellt ist.

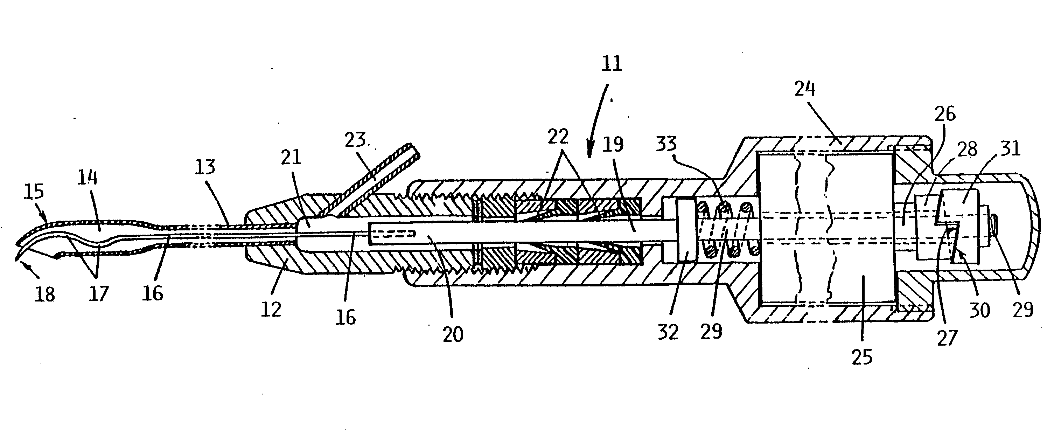

Die Zeichnung zeigt einen Längsschnitt durch das erfindungsgemäß ausgebildete Gerät. Dieses besteht aus einem Handstück 11, an dessen vorderen Teil 12 ein Röhrchen 13 koaxial befestigt ist. Das vordere Ende 14 des Röhrchens 13 ist etwas flach gedrückt und mit einer Krümmung 15 versehen. In dem Röhrchen 13 ist ein axial oszillierend bewegbarer Draht 16 mit relativ viel Spiel gelagert, der an seinem vorderen Ende 17 wellenförmig gebogen und mit einer Spitze 18 versehen ist.

Der Draht 16 ist mit seinem hinteren Ende mit einem Schaft bzw. Kolben 19 verbunden, der mit seinem vorderen Ende 20 in die Kammer 21 im Handstück 11 hineinragt, die nach hinten gegen den Kolben 21 mittels Dichtungsmanschetten 22 abgedichtet ist. In die Kammer 21 mündet ein am Handstück angeordneter Anschlußstutzen 23, auf den ein Schlauch aufsteckbar ist, um unter vorbestimmtem Druck stehende Flüssigkeit in die Kammer 21 zu fördern, die dann während der Benutzung des Geräts durch das Drahtführungsröhrchen 13 hindurch und an dessen vorderen Ende austritt, um einen bestimmten hydrostatischen Druck in der eröffneten Vorderkammer des zu operierenden Auges aufrechtzuerhalten, was die Operation im eröffneten Auge erheblich erleichtert bzw. begünstigt.

Mit der Spitze 18 am vorderen Ende des Drahtes 16 wird die Linsenkapselvorderwand entlang einer vom Operateur bestimmten Linie, zumeist eines Kreises, perforiert, wobei die Einstiche dicht bei dicht nebeneinander liegen. Dies wird dadurch erreicht, daß der Draht 16 mit einer bestimmten Frequenz, beispielsweise von etwa 30-80 Hz, hin- und herbewegt wird. Als Antriebsaggregat kann sowohl ein Elektromagnet, als auch ein Elektromotor Anwendung finden, vorausgesetzt, daß die Bewegungscharakteristik eine solche ist, daß die Spitze 18 des oszillierenden Drahtes 16 bei ihrem Vortrieb sicher die Kapselwand zu durchdringen vermag. Die axialen Bewegungen des Drahtes 16 sollen nicht einer Sinusfunktion folgen, da dessen Spitze 18 an den Bewegungsumkehrpunkten die geringsten Geschwindigkeits- und Beschleunigungswerte aufweist. Da die Linsenkapsel aus einem außerordentlich zähen und widerstandsfähigen Gewebe besteht, muß, um gute Arbeitsergebnisse zu erzielen, die Drahtspitze 18 sehr spontan vorgeschnellt werden, um so die Linsenkapselwand schlagartig zu durchstoßen.

Um dies zu erreichen, findet vorzugsweise ein Antriebsaggregat Anwendung, welches aus einem Antriebsmotor mit umlaufendem Rotor und einer relativ einfachen Mechanik besteht, die den Kolben 21 und damit den Draht 16 zurückzieht und dabei gleichzeitig eine diese beaufschlagende Feder spannt, die bei ihrer plötzlichen Entspannung den Draht 16 in gewünschter Weise vorschnellen läßt.

Am hinteren Ende des Handstücks 11 befindet sich in einem Gehäuse 24 ein elektrisches, pneumatischer oder hydraulischer Antriebsmotor 25, dessen Hohlwelle 26 einen mit einer Kurvenbahn 27 versehenen Antriebsring 28 in Drehung versetzt. An diesem liegt die mit dem Antriebsschaft 29 verbundene, gleichfalls mit einer korrespondierenden Kurvenbahn 30 versehene Antriebsscheibe 31 an, die bei einer Drehung des Antriebsringes 28 axial zurückbewegt wird, wobei sie den Antriebsschaft 29 zurückzieht. An diesem befindet sich ein Federteller 32, durch den die Feder 33 gespannt wird. Bei entsprechender Lage des Antriebsrings 28 gegenüber der Antriebsscheibe 31 löst sich letztere von dem Antriebsring 28, sodaß dann die Feder 33 den Antriebsschaft 29 schlagartig nach vorn treiben kann.

**Patentansprüche**

1. Gerät zum Perforieren der Linsenkapselvorderwand im Auge von Lebewesen zwecks Entfernens eines Teilstücks der Kapselwand und Herstellung einer Öffnung, durch welche hindurch der Linsenkapselinhalt entfernbar ist, mit einem an einem Handstück (11) angeordneten Röhrchen (13), dadurch gekennzeichnet, daß im Röhrchen (13) ein an seinem freien Ende mit einer Spitze (18) versehener Draht (16) geführt ist, welcher mit einem diesen in axial hin- und hergehende Bewegungen versetzenden Antriebsmotor (25) verbunden ist.

2. Gerät nach Anspruch 1, dadurch gekennzeichnet, daß die lichte Weite des Röhrchens (13) mindestens etwa doppelt so groß ist wie der Durchmesser des Drahtes (16).

3. Gerät nach Anspruch 1 oder 2, dadurch gekennzeichnet, daß das Röhrchen (13) im Bereich seiner Mündung einen im wesentlichen ovalen Querschnitt aufweist.

4. Gerät nach Anspruch 1, 2 oder 3, dadurch gekennzeichnet, daß der Draht (16) an seinem vorderen freien Ende (17) gegen seine Spitze (18) hin in einer Radialebene zunächst nach der einen Seite und dann nach der anderen, entgegengesetzten Seite gekrümmt ist.

5. Gerät nach einem oder mehreren der Ansprüche 1 bis 4, dadurch gekennzeichnet, daß in das den Draht (16) führende Röhrchen (13) bzw. in das mit diesem verbundene Anschlußstück ein Stutzen (23) zum Anschluß einer Flüssigkeitszuführleitung einmündet.

6. Gerät nach einem oder mehreren der Ansprüche 1 bis 5, dadurch gekennzeichnet, daß der Innenraum im Drahtführungsröhrchen bzw. in dessen Anschlußstück (21) gegen denjenigen im Handstück (11) abgedichtet und ein mit dem Draht verbundener Kolben (19) od. dgl. durch die Dichtung (22) hindurchgeführt ist.

7. Gerät nach Anspruch 6, dadurch gekennzeichnet, daß als Dichtung (22) wenigstens eine Manschettendichtung mit Dichtungslippe vorgesehen ist.

8. Gerät nach einem oder mehreren der Ansprüche 1 bis 7, dadurch gekennzeichnet, daß als Antriebsmotor (25) des mit dem Draht (16) verbundenen Kolbens (19) ein Elektromagnet dient.

9. Gerät nach einem oder mehreren der Ansprüche 1 bis 7, dadurch gekennzeichnet, daß als Antriebsmotor (25) ein Elektromotor dient, welcher mittels eines mechanischen Getriebes periodisch eine auf den mit dem Draht (16) verbundenen Kolben (19) wirkende Feder (33) spannt und diese dann plötzlich wieder entspannt.

10. Gerät nach einem oder mehreren der Ansprüche 1 bis 7, dadurch gekennzeichnet, daß als Antriebsmotor (25) ein pneumatischer oder hydraulischer Zylinder dient, welcher periodisch eine auf den mit dem Draht (16) verbundenen Kolben (19) wirkende Feder (33) spannt und diese dann plötzlich wieder entspannt.

**Claims**

1. Instrument for perforating the anterior wall of the crystalline lens capsule in the eye of living creatures in order to remove a section of the capsule wall and to produce an aperture through which the contents of the crystalline lens capsule can be withdrawn, said instrument having a small tube (13) mounted to a handpiece (11), characterised in that said small tube (13) carries a wire-rod (16) which, at its free end, is provided with a point (18), said wire-rod being connected to a driving motor (25) which imparts axially reciprocating movements to the same.

2. Instrument according to Claim 1, characterised in that the inside width of said small tube (13) is at least about twice as large as the diameter of said wire-rod (16).

3. Instrument according to Claim 1 or 2, characterised in that the small tube (13), in the region of its orifice, is of substantially oval cross section.

4. Instrument according to Claim 1, 2 or 3, characterised in that the wire-rod (16), at its free front end (17), is bent towards its points (18) in a radial plane at first to one side and then to the other side opposite thereto.

5. Instrument according to one or several of Claims 1 to 4, characterised in that a pipe connection (23) for the junction of a liquid feed pipe opens into the small tube (13) which carries said wire-rod (16) and into the connecting piece, respectively, which is joined thereto.

6. Instrument according to one or several of Claims 1 to 5, characterised in that the interior of said small tube which carries the wire-rod and of the connecting pipe (21) thereof, respectively, is sealed from that of the handpiece (11) and in that a piston (19) or the like which is connected to the wire-rod is passed through the seal (22).

7. Instrument according to Claim 6, characterised in that at least one gasket having a sealing lip is provided as seal (22).

8. Instrument according to one or several of Claims 1 to 7, characterised in that an electromagnet serves as driving motor (25) of the piston (19) connected to the wire-rod (16).

9. Instrument according to one or several of Claims 1 to 7, characterised in that an electric motor serves as driving motor (25), said electric motor, by way of a mechanical gearing, periodically tensioning a spring (33) acting on the piston (19) connected to said wire-rod (16) and then suddenly untensioning the same again.

10. Instrument according to one or several of Claims 1 to 7, characterised in that a pneumatic or hydraulic cylinder serves as driving motor (25), said cylinder periodically tensioning a spring (33) acting on the piston (19) connected to said wire-rod (16) and then suddenly untensioning the same again.

**Revendications**

1. Instrument pour perforer la paroi antérieure de la capsule du cristallin dans l'œil des êtres vivants, afin d'enlever une section de la paroi de la capsule et de produire une ouverture, à travers laquelle le contenu de la capsule du cristallin peut être retiré, ledit instrument comprenant un tuyau étroit (13) monté à une pièce à main (11), caractérisé en ce que, dans ledit tuyau étroit (13), un fil (16) est porté, qui, à son extrémité libre, est pourvu d'une pointe (18), ledit fil étant relié à un moteur de commande (25) qui impose des mouvements axialement réciproques à celui-ci.

2. Instrument selon revendication 1, caractérisé en ce que le diamètre intérieur du tuyau étroit (13) est au moins deux fois si large que le diamètre du fil (16).

3. Instrument selon revendication 1 ou 2, caractérisé en ce que le tuyau étroit (13), dans la région de son orifice, présente une section transversale sensiblement ovale.

4. Instrument selon revendication 1, 2 ou 3, caractérisé ce en ce que le fil (16), à son extrémité frontale libre (17), est courbé vers sa pointe (18) sur un plan radial d'abord à un côté et puis à l'autre côté opposé.

5. Instrument selon l'une ou plusieurs des revendications 1 à 4, caractérisé en ce qu'une tubulure (23) pour la jonction d'un conduit d'alimentation à liquides débouche dans le tuyau étroit (13) portant le fil (16), respectivement dans la pièce de jonction qui est reliée à celui-ci.

6. Instrument selon l'une ou plusieurs des revendications 1 à 5, caractérisé en ce que l'intérieur du tuyau étroit portant le fil, respectivement de la pièce de jonction (21) de celui-ci, est étanchée contre l'intérieur de la pièce à main (11) et qu'un piston (19) ou autre chose pareille, étant relié au fil, est passé à travers le joint (22).

7. Instrument selon revendication 6, caractérisé en ce qu'au moins une garniture à bec étanchant est prévue comme joint (22).

8. Instrument selon l'une ou plusieurs des revendications 1 à 7, caractérisé en ce qu'un électro-aimant sert de moteur de commande (25) du piston (19) relié au fil (16).

9. Instrument selon l'une ou plusieurs des revendications 1 à 7, caractérisé en ce qu'un électro-moteur sert de moteur de commande (25), ledit électro-moteur, au moyen d'un engrenage mécanique, périodiquement bandant un ressort (33) opérant sur le piston (19) relié au fil (16), et puis soudainement débandant celui-ci de nouveau.

10. Instrument selon l'une ou plusieurs des revendications 1 à 7, caractérisé en ce qu'un cylindre pneumatique ou hydraulique sert de moteur de commande (25), ledit cylindre périodiquement bandant un ressort (33) opérant sur le piston (19) relié au fil (16), et puis soudainement débandant celui-ci de nouveau.

0 170 650